# FASCICULE DE BREVET EUROPEEN

(11) **EP 1 458 369 B1**
(45) Date de publication et mention de la délivrance du brevet: **06.12.2006**
(21) Numéro de dépôt: 02799797.2
(22) Date de dépôt: 09.12.2002
(51) Int. Cl.: A61K 31/07, A61K 31/19, A61K 31/20, A61P 17/00, A61Q 19/00, A61Q 5/00

(54) **GEL COMPRENANT AU MOINS UN RETINOIDE ET DU PEROXYDE DE BENZOYLE**
GEL MIT MINDESTENS EINEM RETINOID UND BENZOYLPEROXID
GEL COMPRISING AT LEAST A RETINOID AND BENZOYL PEROXIDE

(30) Priorité: 21.12.2001 FR 0116747
(43) Date de publication de la demande: 22.09.2004
(73) Titulaire: Galderma Research & Development, S.N.C., 06560 Valbonne, Sophia Antipolis (FR)
(72) Inventeur: ORSONI, Sandrine, F-06210 Mandelieu (FR); WILLCOX, Nathalie, 06460 Saint Vallier de Thiey (FR)
(74) Mandataire: Bernstein, Claire Jacqueline
(86) Numéro de dépôt international: PCT/FR2002/004233
(87) Numéro de publication internationale: WO 2003/055472

(56) Documents cités:
- EP-A- 0 335 115
- WO-A-93/07856
- FR-A- 2 378 523
- US-A- 4 189 501
- US-A- 4 792 452
- D.CARON E.A.: "Skin tolerance of adapalene 0.1% gel in combination with other topical antiacne treatments" JOURNAL OF THE AMERICAN ACADEMY OF DERMATOLOGY, vol. 36, no. 6,2, 1997, pages S113-S115, XP000974553
- B.MARTIN E.A.: "Chemical stability of adapalene and tretinoin when combined with benzoyl peroxide in presence and in absence of visible light and ultraviolet radiation" THE BRITISH JOURNAL OF DERMATOLOGY, vol. 139, no. S52, 1998, pages 8-11, XP008007635
- S.HURWITZ: "The combined effect of vitamin A acid and benzoyl peroxide in the treatment of acne" CUTIS: CUTANEOUS MEDICINE FOR THE PRACTITIONER, vol. 17, no. 3, 1976, pages 585-590, XP008007633

## Description

L'invention se rapporte à une composition comprenant, dans un milieu physiologiquement acceptable, au moins un rétinoide, du peroxyde de benzoyle dispersé et au moins un gélifiant pH-indépendant.

L'utilisation de plusieurs classes de principes actifs est un outil thérapeutique auquel il est fréquemment fait recours, notamment pour le traitement de désordres dermatologiques. En effet, il est connu d'utiliser dans le traitement des dermatites, des corticostéroïdes comme par exemple l'hydrocortisone, le miconazole ou le valérate de betaméthasone, des antihistaminiques (ex. mizolastine) et/ou des agents kératolytiques comme l'acide salicylique. Différents antifongiques comme les dérivés allylamines, les triazoles, les antibactériens ou antimicrobiens comme par exemple les antibiotiques, les quinolones et les imidazoles, sont également classiquement associés dans le traitement de maladies dermatologiques. Les peroxydes, les vitamines D et les rétinoides sont également décrits pour le traitement topique de diverses pathologies liées à la peau ou les muqueuses, en particulier l'acné.
La combinaison de plusieurs traitements locaux (antibiotiques, rétinoides, peroxydes, zinc) est également utilisée en dermatologie pour permettre d'augmenter l'efficacité des principes actifs et de diminuer leur toxicité (Cunliffe W.J., *J*. *Dermatol. Treat.,* 2000, 11 (suppl2), S13-S14).
L'application multiple de différents produits dermatologiques peut être assez lourde et astreignante pour le patient.
On comprend donc l'intérêt de chercher à obtenir un nouveau traitement efficace sur les affections dermatologiques dans une composition stable offrant une bonne cosméticité, permettant une application unique et une utilisation agréable pour le patient.
Parmi cette panoplie de thérapeutiques proposée à l'homme du métier, rien ne l'encourageait à associer, dans la même composition, le peroxyde de benzoyle et un rétinoide.

Toutefois, la formulation d'une telle composition pose plusieurs problèmes.

Tout d'abord, l'efficacité du peroxyde de benzoyle est liée à sa décomposition lorsqu'il est mis en contact avec la peau. En effet, ce sont les propriétés oxydantes des radicaux libres produits lors de cette décomposition qui conduisent à l'effet désiré. Aussi, afin de maintenir au peroxyde de benzoyle une efficacité optimale, il est important de prévenir sa décomposition avant utilisation, c'est à dire durant le stockage.

Or le peroxyde de benzoyle est un composé chimique instable qui rend difficile sa formulation dans des produits finis.

La solubilité et la stabilité du peroxyde de benzoyle ont été étudiées par Chellquist *et al.* dans l'éthanol, le propylène glycol et différents mélanges de polyéthylène glycol 400 (PEG 400) et d'eau (Chellquist E.M. et Gorman W.G., *Pharm. Res.,* 1992, Vol 9: 1341-1346). Le peroxyde de benzoyle est particulièrement soluble dans le PEG400 et l'éthanol comme le montre le tableau suivant :

| **Solvant** | **Solubilité du peroxyde de benzoyle (mg/g)** |
|---|---|
| PEG 400 | 39.6 |
| Ethanol | 17.9 |
| Propylène Glycol | 2.95 |
| Propylène Glycol / Eau (75 :25) | 0.36 |
| Glycérine | 0.15 |
| Eau | 0.000155 |

Ce document précise par ailleurs que la stabilité du peroxyde de benzoyle est fortement influencée par la composition chimique de la formulation et par la température de stockage. Le peroxyde de benzoyle est extrêmement réactif et se dégrade en solution à basse température en raison de l'instabilité de sa liaison peroxyde.
Les auteurs constatent ainsi que le peroxyde de benzoyle en solution se dégrade plus ou moins rapidement dans tous les solvants étudiés en fonction du type de solvant et de sa concentration.
Les temps de dégradation du peroxyde de benzoyle dans le PEG 400 (0.5 mg/g), dans l'éthanol et dans le propylène glycol sont respectivement de 1,4 , 29 et 53 jours à 40°C.
Une telle dégradation ne permet pas la préparation d'un produit destiné à la vente.

II est connu par ailleurs que le peroxyde de benzoyle est plus stable dans l'eau et le propylène glycol lorsqu'il est en suspension (ie sous forme dispersée), puisqu'il n'est pas dégradé après 90 jours de conservation dans ces solvants.
Ainsi, pour limiter le problème d'instabilité rapide du peroxyde de benzoyle en solution, il s'est avéré avantageux de formuler le peroxyde de benzoyle sous forme dispersée.
Cependant, ce type de formulation n'est pas totalement satisfaisante dans la mesure où on constate toujours une dégradation du peroxyde de benzoyle dans le produit fini.

Une autre difficulté à surmonter pour la préparation d'une composition comprenant à la fois du peroxyde de benzoyle et un rétinoide est que la plupart des rétinoides est particulièrement sensible à l'oxydation naturelle, à la lumière visible et aux ultra-violets, et le peroxyde de benzoyle étant un oxydant fort, la compatibilité chimique de ces composés dans une même formulation pose de nombreux problèmes de stabilité du point de vue physique et chimique.

Une étude de stabilité de deux rétinoides a été réalisée en combinant deux produits commercialisés, l'un contenant un rétinoide (trétinoine ou adapalene) et le second à base de peroxyde de benzoyle *(B. Martin et al., Br.J.Dermatol. (1998) 139, (suppl.52), 8-11).*
La présence de la formulation à base de peroxyde de benzoyle provoque une dégradation très rapide des rétinoides sensibles à l'oxydation : on mesure que 50% de la trétinoine se dégrade en 2 heures, et 95% en 24 heures. Dans la composition dans laquelle le rétinoide est l'adapalene, aucune dégradation de l'adapalène n'a été mesurée pendant 24 heures. Cette étude confirme que le peroxyde de benzoyle se dégrade et dégrade les rétinoides sensibles à l'oxydation au cours du temps en relarguant progressivement de l'acide benzoïque dans des produits finis.
Par contre, aucune indication n'est faite quant à la stabilité physique des deux compositions mises en présence, ni sur l'activité thérapeutique susceptible d'être obtenue *in fine* par la combinaison des deux principes actifs dans la même composition.
Rien n'incitait à associer ces deux agents actifs afin d'obtenir une composition stable de type gel sachant qu'il était usuellement connu que la présence du peroxyde de benzoyle déstabilisait chimiquement et physiquement ce type de composition.

Or il est clair que la dégradation du peroxyde de benzoyle et des rétinoides n'est pas souhaitable dans la mesure où elle nuit à l'efficacité de la composition les contenant.

Par ailleurs, un produit fini, en particulier lorsqu'il s'agit de compositions pharmaceutiques
ou cosmétiques, doit conserver tout au long de sa durée de vie, des critères physico-chimiques précis permettant de garantir sa qualité pharmaceutique ou cosmétique, respectivement. Parmi ces critères, il est nécessaire que les propriétés rhéologiques soient conservées. Elles définissent le comportement et la texture de la composition lors de l'application, mais aussi les propriétés de libération du principe actif [Rapport commission SFSTP 1998] et l'homogénéité du produit lorsque les principes actifs y sont présents à l'état dispersé.

En particulier, la formulation en gel du peroxyde de benzoyle et d'un rétinoide est avantageuse pour les traitements topiques, tels que celui de l'acné, car elle évite notamment de laisser subsister un toucher gras sur la peau.
Or, une autre difficulté à surmonter pour la préparation d'une composition comprenant notamment du peroxyde de benzoyle, lorsqu'elle se trouve sous forme de gel, est que les gélifiants sont déstabilisés par l'acide benzoïque libéré lors de la dégradation du peroxyde de benzoyle.
En effet, les agents épaississants les plus couramment utilisés pour la formulation de ces compositions avec du peroxyde de benzoyle sont les polymères d'acide acrylique (Carbomer) et les celluloses seules ou associées à des silicates.
Or, l'utilisation de carbomères dans des compositions de type gel aqueux ne donne pas de bons résultats en terme de stabilité chimique du peroxyde de benzoyle et en terme de stabilité rhéologique. Comme décrit par Bollinger (Bollinger, Journal of Pharmaceutical Science, 1977, vol 5), il a été observé une perte de 5 à 20% de peroxyde de benzoyle au bout de 2 mois à 40°C selon le neutralisant du carbomère utilisé. De plus, la libération d'acide bonzoïque provoque la dépolymérisation des carbomères, donnant une chute de viscosité pouvant entraîner un déphasage.
Dans d'autres gels constitués d'un mélange d'hydroxypropylcellulose et de silicate d'aluminium et de magnésium, on observe également une chute de viscosité au cours du temps qui entraîne une sédimentation des actifs en suspension et une hétérogénéité de la dispersion dans te produit fini. De tels gels sont notamment illustrés par la demande FR 2378523.
Cette instabilité des gels de peroxyde de benzoyle nuit à leur efficacité et à leur cosméticité,

Il reste donc le besoin de disposer d'une composition gélifiée physiquement stable contenant du peroxyde de benzoyle et un rétinoine.
Or, la Demanderesse a réalisé de façon surprenante une composition satisfaisant ce besoin, qui comprend du peroxyde de benzoyle dispersé, libre ou encapsulé, au moins un rétinoide et un gélifiant pH-Indépendant ayant une bonne stabilité physique, c'est-à-dire ne présentant pas de chute de viscosité dans le temps et à des températures comprises entre 4 et 40°C, et maintenant une bonne stabilité chimique des deux actifs (peroxyde de benzoyle et rétinoide), c'est-à-dire qu'on ne constate pas de dégradation des actifs dans le temps et à des températures comprises entre 4 et 4Q°C.
La Demanderesse a également découvert de manière surprenante que l'on pouvait obtenir une parfaite dispersion des principes actifs en suivant un procédé de préparation particulier. Ce procédé de préparation réalisé à froid permet d'obtenir une granulométrie optimale et une dispersion homogène des deux actifs dans la composition, tout en garantissant la stabilité physique du produit.

L'invention se rapporte donc à une composition comprenant, dans un milieu physiologiquement acceptable, au moins un rétinoide, du peroxyde de benzoyle dispersé et au moins un gélifiant pH-indépendant.

La composition selon l'invention est de préférence sous la forme d'un gel aqueux.

On entend par gel aqueux, une composition contenant, dans une phase aqueuse, une masse visco-élastique formée à partir de suspensions colloïdates (gélifiant).

Par gélifiant pH indépendant, on entend un gélifiant capable de conférer une viscosité suffisante à la composition pour maintenir en suspension le rétinoïde et le peroxyde de benzoyle, même sous l'influence d'une variation de pH due au relarguage d'acide benzoïque par le peroxyde de benzoyle.

Ces gélifiant pH imdépendant sont les gélifiants de la famille des polyacrylamides tels que le mélange Sodium acryloyldimethyltaurate copolymer / isohexadecane / polysorbate 80 vendu sous le nom Simulgel 600 par la société Seppic, le mélange polyacrylamide / isoparaffine C13-14 / laureth-7 comme, par exemple, celui vendu sous le nom de Sepigel 305 par la société Seppic, la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propytèneglycol (39%) et d'eau (26%)), la famille des amidons modifiés tels que l'amidon de pomme de terre modifié vendu sous le nom de Structure Solanace ou bien leurs mélanges.

Les gélifiants préférés sont issus de la famille des polyacrylamides tel que le Simulgel 600 ou le Sepigel 305 ou leurs mélanges.

Le gélifiant tel que décrit ci-dessus peut être utilisé aux concentrations préférentielles allant de 0,1 à 15 % et, plus préférentiellement, allant de 0,5 à 5 %.

La composition selon l'invention contient au moins un rétinoide.

Par rétinoide, on entend tout composé se liant aux récepteurs RAR et/ou RXR.

Préférentiellement, le rétinoide est un composé choisi dans la famille des rétinoides benzonaphtaleniques tels que décrits dans la demande de brevet EP 0 199 636. En particulier, on préférera l'adapalène.

D'autres rétinoides peuvent être choisis parmi ceux décrits dans les brevets ou demandes de brevet suivants : US 4,666,941, US 4,581,380, EP 0 210 929, EP 0 232 199, EP 0 260162, EP 0 292 348, EP 0 325 540, EP 0 359 621, EP 0 409 728, EP 0 409 740, EP 0 552 282, EP 0 584191, EP 0 514 264, EP 0 514 269, EP 0 661 260, EP 0 661 258, EP 0 658 553, EP 0 679 628, EP 0 679 631, EP 0 679 630, EP 0 708 100, EP 0 709 382, EP 0 722 928, EP 0 728 739, EP 0 732 328, EP 0 740 937, EP 0 776 885, EO 0 776 881, EP 0 823 903, EP 0 832 057, EP 0 832 081, EP 0 816 352, EP 0 826 657, EP 0 874 626, EP 0 934 295, EP 0 915 823, EP 0 882 033, EP 0 850 909, EP 0 879 814, EP 0 952 974, EP 0 905118, EP 0 947 496, WO98/56783, WO99/10322, WO99/50239, WO99/65872.

Bien entendu, la quantité des deux agents actifs, peroxyde de benzoyle et rétinoide, dans la composition selon l'invention dépendra de l'association choisie et donc particulièrement du rétinoide considéré et de la qualité du traitement désiré.

Les concentrations en rétinoide préférées sont comprises entre 0,0001 et 20 % en poids par rapport au poids total de la composition.

Le peroxyde de benzoyle pourra aussi bien être utilisé sous la forme libre ou bien sous une forme encapsulée sous forme adsorbée sur, ou absorbée dans, tout support poreux. Il peut s'agir par exemple de peroxyde de benzoyle encapsulé dans un système polymérique constitué de microsphères poreuses, comme par exemple des microéponges vendues sous le nom de Microsponges P009A Benzoyle peroxyde par la société Advanced Polymer System.

Pour donner un ordre de grandeur, la composition selon l'invention comprend avantageusement entre 0,0001 et 20 % en poids de peroxyde de benzoyle et entre 0,0001 et 20 % en poids de rétinoide par rapport au poids total de la composition, et de préférence, respectivement, entre 0,025 et 10 % en poids de peroxyde de benzoyle et entre 0,001 et 10 % en poids de rétinoide par rapport au poids total de la composition.

Par exemple, dans les compositions pour le traitement de l'acné, le peroxyde de benzoyle est utilisé, de préférence, à des concentrations allant de 2 à 10 % en poids et plus particulièrement de 2,5 à 5 % en poids par rapport au poids total de la composition. Le rétinoide est quant à lui utilisé dans ce type de composition à des concentrations allant généralement de 0,05 à 1 % en poids par rapport au poids total de la composition.

De façon avantageuse, la granulométrie du rétinoide et du peroxyde de benzoyle est telle qu'au moins 80% en nombre des particules, et de préférence au moins 90% en nombre des particules, ont un diamètre inférieur à 25 µm et au moins 99% en nombre des particules ont un diamètre inférieur à 100 µm.

Selon l'invention, d'une manière avantageuse, le gel contenant le peroxyde de benzoyle et un rétinoide comprend au moins de l'eau et peut également comprendre un agent propénétrant et/ou un tensioactif liquide mouillant.

Les compositions de l'invention peuvent contenir un ou plusieurs agents propénétrants dans des concentrations préférentielles allant de 0 à 20 % et plus préférentiellement allant de 2 à 6 % en poids par rapport au poids total de la composition. Ils doivent être généralement non solubilisants des actifs au pourcentage utilisé, ne pas provoquer de réactions exothermiques néfastes pour le peroxyde de benzoyle, aider à la bonne dispersion des actifs et avoir des propriétés anti-mousse. Parmi les agents propénétrants, on utilise préférentiellement des composés tels que le propylène glycol, le dipropylène glycol, le propylène glycol dipélargonate, le lauroglycol, l'éthoxydiglycol.

L'agent propénétrant particulièrement préféré est le propylène glycol.

Avantageusement, les compositions selon l'invention peuvent contenir également un ou plusieurs agents tensioactifs liquides mouillants dans des concentrations préférentielles allant de 0 à 10 % et plus préférentiellement allant de 0,1 à 2 %. Le pouvoir mouillant est la tendance d'un liquide à s'étaler sur une surface.
De préférence, il s'agit de tensioactifs ayant une HLB (Hydrophilic Lipophilic Balance) de 7 à 9, ou bien des tensioactifs non ioniques de type copolymères polyoxyéthylénés et/ou polyoxypropylénés. Ils doivent être liquides de manière à s'incorporer aisément dans la composition sans qu'il soit nécessaire de la chauffer.

Parmi les agents mouillants, on utilise préférentiellement des composés de la famille des Poloxamers et plus particulièrement le Poloxamer 124 et/ou le Poloxamer 182.

L'agent tensioactif liquide mouillant particulièrement préféré est le Poloxamer 124.

La composition peut comprendre en outre tout additif usuellement utilisé dans le domaine cosmétique ou pharmaceutique, tel que des séquestrants, des antioxydants, des filtres solaires, des conservateurs, des charges, des électrolytes, des humectants, des colorants, de bases ou d'acides usuels, minéraux ou organiques, des parfums, des huiles essentielles, des actifs cosmétiques, des hydratants, des vitamines, des acides gras essentiels, des sphingolipides, des composés auto-bronzants tels que la DHA, des agents apaisants et protecteurs de la peau tels que l'allantoine. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0 à 20 % en poids par rapport au poids total de la composition.

On peut citer comme exemple d'agents séquestrants, l'acide éthylènediamine tétracétique (EDTA), ainsi que ses dérivés ou ses sels, la dihydroxyethylglycine, l'acide citrique, l'acide tartrique, ou leurs mélanges.

On peut citer comme exemples de conservateurs le chlorure de benzalkonium, le phénoxyéthanol, l'alcool benzylique, la diazolidinylurée, les parabens, ou leurs mélanges.

On peut citer comme exemples d'agents humectants, la glycérine et le sorbitol.

En particulier, l'invention concerne également une composition pharmaceutique ou cosmétique pour application topique sur la peau, les phanères ou les muqueuses, sous forme d'un gel aqueux, caractérisée en ce qu'elle contient, dans un milieu physiologiquement acceptable et compatible avec l'application topique sur la peau, les phanères ou les muqueuses, une phase active comprenant (exprimé en pourcentage en poids) :
- 0 à 90 %, préférentiellement 5 à 25 %, notamment 10 à 20 %, d'eau ;
- 0 à 10 %, préférentiellement 0 à 2 %, notamment 0 à 0,5 %, de tensioactif liquide mouillant ;
- 0 à 20 %, préférentiellement 0 à 10 %, notamment 2 à 5 %, de propénétrant ;
- 0,0001 à 20 %, préférentiellement 0,025 à 10 %, de peroxyde de benzoyle ;
- 0,0001 à 20 %, préférentiellement 0,001 à 10 %, de rétinoide ;
- et une phase aqueuse comprenant un gélifiant pH-indépendant, et de l'eau.

La phase aqueuse de l'émulsion selon l'invention peut comprendre de l'eau, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène ou l'eau d'Aix les Bains.

Ladite phase aqueuse peut être présente à une teneur comprise entre 10 et 90 % en poids par rapport au poids total de la composition, de préférence comprise entre 20 et 80 % en poids.

Une composition préférée selon l'invention comprend dans l'eau :
- 2,50 % de peroxyde de benzoyle,
- 0,10 % d'adapalene,
- 0,10 % d'EDTA disodique,
- 4,00 % de glycérine,
- 4,00 % de propylène glycol,
- 0,05 %de docusate de sodium,
- 0,20 % de Poloxamer 124,
- 4,00 % de sodium acryloyldimethyltaurate copolymer & isohexadecane & polysorbate 80,
- qs pH 5 d'hydroxyde de sodium.

La présente invention a aussi pour objet la composition telle que décrite précédemment à titre de médicament.

L'invention se rapporte également à l'utilisation de la nouvelle composition telle que décrite précédemment en cosmétique et en dermatologie.

L'invention a également pour objet un procédé de préparation d'une composition de type gel aqueux comprenant l'élaboration d'une phase active, d'une phase aqueuse, et d'une phase de gélification, à température ambiante (TA), c'est à dire entre 20 et 25°C, et comprenant successivement les étapes suivantes :
a) la préparation d'une phase aqueuse comprenant de l'eau et éventuellement un chélatant et/ou un propénétrant et/ou un humectant ;
b) la préparation d'une phase active comprenant le mélange dans l'eau du rétinoide, du peroxyde de benzoyle et, éventuellement, d'un tensioactif liquide mouillant et/ou d'un propénétrant sous agitation ;
c) l'introduction de la phase active dans la phase aqueuse, sous agitation ; et
d) l'introduction du gélifiant sous agitation dans le mélange issu de l'étape c).

Dans une variante de réalisation, le procédé de préparation de la composition de gel aqueux comprenant l'élaboration d'une phase active, d'une phase aqueuse, et d'une phase de gélification, à température ambiante comprend successivement les étapes suivantes :
a) la préparation d'une phase aqueuse comprenant de l'eau et éventuellement un chélatant et/ou un propénétrant et/ou un humectant ;
b) la préparation de deux phases actives, l'une comprenant le mélange dans l'eau du rétinoide, l'autre comprenant le mélange dans l'eau du peroxyde de benzoyle et, éventuellement, d'un tensioactif liquide mouillant et/ou d'un propénétrant sous agitation ;
c) l'introduction des phases actives dans la phase aqueuse, sous agitation ; et
d) l'introduction du gélifiant sous agitation dans le mélange issu de l'étape c).

La préparation du gel aqueux suivant l'un de ces procédés opératoires s'est avéré apporter beaucoup d'avantages par rapport à la préparation d'autres gels aqueux déjà connus, notamment car elle est plus simple et que l'incorporation du gélifiant à la fin du procédé permet d'obtenir une meilleure dispersion des particules par emprisonnement, ces gels peuvent également être filmogènes donc limiter la perspiration. Au moins 80 % en nombre des particules et de préférence au moins 90 % en nombre des particules ont un diamètre inférieur à 25 µm et au moins 99 % en nombre des particules ont un diamètre inférieur à 100 µm dans la composition.

De part l'activité kératolytique, bactéricide et anti-inflammatoire du peroxyde de benzoyle et l'activité marquée des rétinoides dans les domaines de la différentiation et de la prolifération cellulaire, les compositions de l'invention conviennent particulièrement bien dans les domaines thérapeutiques suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle, l'hidradenite supurative,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore fatopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation telles que les folliculites,
4) pour traiter toutes les proliférations dermiques ou épidermiques qu'elles soient bénignes ou malignes, qu'elles soient ou non d'origine virale telles que verrues vulgaires, les verrues planes, le molluscum contagiosum, et l'épidermodysplasie verruciforme, les papillomatoses orales ou florides et les proliférations pouvant être induites par les ultra-violets notamment dans le cas des kératoses actiniques,
5) pour réparer ou lutter contre le vieillissement de la peau, qu'il soit photo-induit ou chronologique, ou pour réduire les pigmentations, ou toutes pathologies associées au vieillissement chronologique ou actinique,
6) pour traiter de manière préventive ou curative les troubles de la cicatrisation, les ulcères cutanés, pour prévenir ou pour réparer les vergetures, ou encore pour favoriser la cicatrisation,
7) pour lutter contre les troubles de la fonction sébacée tels que l'hyperséborrhée de l'acné ou la séborrhée simple,
8) dans le traitement de toute affection d'origine fongique au niveau cutané tel que le tinea pedis et le tinea versicolor,
9) dans le traitement d'affections dermatologiques à composante immunologique,
10) dans le traitement de désordres cutanés dus à une exposition aux rayonnements U.V., et
11) dans le traitement d'affections dermatologiques liées à une inflammation ou une infection des tissus environnants le follicule pileux, notamment dues à une colonisation ou infection microbienne notamment l'impétigo, la dermite seborrhéique, la folliculite, le sycosis barbae ou impliquant tout autre agent bactérien ou fongique.

Les compositions selon l'invention sont particulièrement adaptées au traitement, de manière préventive ou curative, des acnés vulgaires.

Un objet de l'invention se rapporte également à la fabrication de préparation pharmaceutique destinée à la prévention ou au traitement des affections dermatologiques liées à des désordres de la différentiation et/ou de la prolifération cellulaire et/ou de la kératinisation, ainsi qu'à la fabrication de préparation pharmaceutique destinée à prévenir ou à traiter les acnés vulgaires.

Les compositions selon l'invention trouvent également une application dans le domaine cosmétique, en particulier pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Les compositions selon l'invention trouvent aussi une application dans l'hygiène corporelle et capillaire.

La présente invention se rapporte ainsi également à l'utilisation cosmétique d'une composition selon l'invention pour le traitement des peaux à tendance acnéique, pour faire repousser les cheveux ou éviter leur chute, pour lutter contre l'aspect gras de la peau ou des cheveux, ou dans le traitement des peaux physiologiquement sèches, ou pour prévenir et/ou lutter contre le vieillissement photo-induit ou chronologique.

Les exemples de formulations ci-dessous permettent d'illustrer les compositions selon l'invention. Des exemples de procédés de préparation des compositions selon l'invention ainsi que des exemples illustrant la stabilité physique et chimique des compositions, sont également décrits.

### I. EXEMPLES DE FORMULATIONS

Dans les compositions ci-après (exemples 1 à 5) les proportions des différents constituants sont exprimés en pourcentage en poids par rapport au poids total de la composition.

### Exemple 1 :

| **Phase** | **Composants** | **%** |
|---|---|---|
| **active** | PEROXYDE DE BENZOYLE | 5.00% |
| | ADAPALENE | 0.10% |
| | DIPROPYLENE GLYCOL | 4.00% |
| | EAU PURIFIEE | 10% |
| | POLOXAMER 182 | 0.20% |
| | | |
| **aqueuse** | EAU PURIFIEE | QS 100% |
| | SILICA | 0.02% |
| | GLYCEROL | 2.00% |
| | MICROSPONGES | 4.00% |
| | | |
| **gélifiant** | POLYACRYLAMIDE (and) C13-14 ISOPARAFFIN (and) LAURETH-7 | 3.50% |

### Exemple 2 :

| **Phase** | **Composants** | **%** |
|---|---|---|
| **active** | PEROXYDE DE BENZOYLE | 5% |
| | ADAPALENE | 0.10% |
| | EAU PURIFEE | 10.00% |
| | PROPYLENE GLYCOL | 2.00% |
| | POLOXAMER 124 | 0.20% |
| | | |
| **aqueuse** | EAU PURIFIEE | **QS 100%** |
| | E.D.T.A DISODIQUE | 0.10% |
| | GLYCEROL | 4.00% |
| | PROPYLENE GLYCOL | 2.00% |
| | DOCUSATE DE SODIUM | 0.05% |
| | | |
| **gélifiant** | SODIUM ACRYLOYLDIMETHYLTAURATE COPOLYMER & ISOHEXADECANE & POLYSORBATE 80 | 4.00% |

### Exemple 3 :

| **Phase** | **Composants** | **%** |
|---|---|---|
| **active** | PEROXYDE DE BENZOYLE | 2.50% |
| | ADAPALENE. | 0.10% |
| | EAU PURIFEE | 10.00% |
| | PROPYLENE GLYCOL | 2.00% |
| | POLOXAMER 124 | 0.20% |
| | | |
| **aqueuse** | EAU PURIFIEE | **QS 100%** |
| | E.D.T.A DISODIQUE | 0.10 % |
| | GLYCERINE | 4.00% |
| | PROPYLENE GLYCOL | 2.00% |
| | DOCUSATE DE SODIUM | 0.05% |
| | | |
| **gélifiant** | SODIUM ACRYLOYLDIMETHYLTAURATE COPOLYMER & ISOHEXADECANE & POLYSORBATE 80 | 4.00% |
| | | |
| **Ajusteur de pH** | HYDROXYDE DE SODIUM | QS PH 5.00 |

### Exemple 4 :

| **Phase** | **Composants** | **%** |
|---|---|---|
| **active** | PEROXYDE DE BENZOYLE | 5.00% |
| | ADAPALENE | 0.10% |
| | EAU PURIFEE | 10.00% |
| | PROPYLENE GLYCOL | 2.00% |
| | POLOXAMER 124 | 0.20% |
| | | |
| **aqueuse** | EAU PURIFIEE | **QS 100%** |
| | E.D.T.A DISODIQUE | 0.10 % |
| | GLYCERINE | 4.00% |
| | PROPYLENE GLYCOL | 2.00% |
| | SODIUM C14-16 OLEFIN SULFONATE | 0.05% |
| | | |
| **gélifiant** | PEG-150/decyl/SMDI copolymer | 3.50% |

### Exemple 5 :

| **Phase** | **Composants** | **%** |
|---|---|---|
| **Active 1** | PEROXYDE DE BENZOYLE | 2.50% |
| | EAU PURIFEE | 20.00% |
| | PROPYLENE GLYCOL | 1.00% |
| | POLOXAMER 124 | 0.10% |
| **Phase Active 2** | ADAPALENE | 0.10% |
| | PROPYLENE GLYCOL | 1.00% |
| | POLOXAMER 124 | 0.10% |
| | | |
| **aqueuse** | EAU PURIFIEE | **QS 100%** |
| | E.D.T.A DISODIQUE | 0.10 % |
| | GLYCERINE | 4.00% |
| | PROPYLENE GLYCOL | 2.00% |
| | DOCUSATE DE SODIUM | 0.05% |
| | | |
| **gélifiant** | SODIUM ACRYLOYLDIMETHYLTAURATE COPOLYMER & ISOHEXADECANE & POLYSORBATE 80 | 4.00% |
| | | |
| **Ajusteur de pH** | HYDROXYDE DE SODIUM | QS PH 5.00 |

### II. EXEMPLES DE PROCEDE DE PREPARATION

### Exemple 6 :

Les exemples de procédé de préparation sont proposés
Il s'agit de la préparation de la composition objet de l'exemple 3 :

| MODE OPERATOIRE | PARAMETRES |
|---|---|
| Dans le bécher (Phase 1) introduire : | T° : TA °c |
| - Eau Purifiée | AGITATEUR : Rayneri |
| - E.D.T.A Disodique | PALE : Défloculeuse |
| - Glycérine | AGITATION : 350 tr/min |
| | TEMPS : 10 min |
| Dans un bêcher annexe introduire : | T° : TA °c |
| - Propylène Glycol | AGITATEUR : Plaque d'agitation |
| - Docusate De Sodium | PALE : Barreau magnétique |
| Mettre sous agitation magnétique jusqu'à parfaite solubilisation du docusate de sodium | TURBINE : N.A |
| | TEMPS : 45 min |
| Phase active : Dans un bécher annexe de taille adaptée introduire : | T° : TA + Bain marie d'eau froide |
| - Propylène Glycol (PG) | AGITATEUR : Disperseur |
| - Poloxamer 124 | PALE : Polytron |
| - Eau Purifiée | AGITATION: 9000 tr/min |
| - Peroxyde de benzoyle | TEMPS : 30 min |
| - Adapalene | |
| Mettre sous agitation Polytron jusqu'à parfaite dispersion des actifs, dans un bain marie d'eau froide pour éviter la surchauffe des actifs. | |
| Lorsque le mélange Docusate De Sodium + PG est parfaitement solubilisé l'incorporer dans la phase 1. | T°: TA °c |
| | AGITATEUR : Rayneri |
| Puis ajouter la phase active . | PALE : Défloculeuse |
| Sous agitation Rayneri jusqu'à obtention d'un mélange homogène. | AGIATION : 400-500 tr/min |
| | TEMPS : 20 min |
| Ajouter en final : | T° : TA °c |
| - Simulgel 600 | AGITATEUR : Rayneri |
| Sous agitation Rayneri le temps nécessaire à une bonne homogénéité du produit fini. | PALE : Défloculeuse |
| | AGITATION : 1000-1200 tr/mn |
| | TEMPS : 25 min |
| Neutralisation : Ajuster le pH qs 5.00+/-0.3 avec : Hydroxyde de sodium à 10% | T° : TA °c |
| | AGITATEUR : Rayneri |
| | PALE: Défloculeuse |
| | AGITATION : 1000-1200 tr/mn |
| | TEMPS : 25 min |

II s'agit de la préparation de la composition objet de l'exemple 5 :

| MODE OPERATOIRE | PARAMETRES |
|---|---|
| Dans le bécher introduire : | T° : TA °c |
| - Eau Purifiée | AGITATEUR : Rayneri |
| - E.D.T.A Disodique | PALE : Défloculeuse |
| - Docusate De Sodium | AGITATION : 350 tr/min |
| - Propylène Glycol | TEMPS : 50 min |
| Mettre sous agitation magnétique jusqu'à parfaite solubilisation du docusate de sodium | |
| Puis introduire : | T° : TA °c |
| - Glycérine | AGITATEUR: Plaque d'agitation |
| | PALE : Défloculeuse |
| | AGITATION : 350 tr/min |
| | TEMPS : 5 min |
| Phase active 1: Dans un bécher annexe de taille adaptée introduire : | T° : TA + Bain marie |
| | d'eau froide |
| - Propylène Glycol (PG) | PALE : Ultra-Turrax |
| - Poloxamer 124 | AGITATION : 13500 tr/min |
| - Eau Purifiée | TEMPS : 30 min |
| - Peroxyde de benzoyle | |
| Mettre sous agitation jusqu'à parfaite dispersion de l'actif, dans un bain marie d'eau froide pour éviter la surchauffe. | |
| Phase active 2: Dans un bécher annexe de taille adaptée introduire : | T° : TA + Bain marie d'eau froide |
| - Propylène Glycol (PG) | PALE : Ultra- Turrax |
| - Poloxamer 124 | AGITATION: 9500 tr/min |
| - Adapalene. | TEMPS : 15 min |
| Incorporer la Phase active 2 dans la phase aqueuse. | T° : TA °c |
| Puis ajouter la phase active 1. | AGITATEUR : Rayneri |
| Sous agitation Rayneri jusqu'à l'obtention d'un mélange homogène. | PALE : Défloculeuse |
| | AGIATION : 300 tr/min |
| | TEMPS: 20 min |
| Ajouter en final: | T° : TA °c |
| - Simulgel 600 | AGITATEUR : Rayneri |
| Sous agitation Rayneri le temps nécessaire à une bonne homogénéité du produit fini. | PALE : Défloculeuse |
| | AGITATION : 680 tr/mn |
| | TEMPS: 35 min |
| Neutralisation : Ajuster le pH qs 5.00+/-0.3 avec : | T° : TA °c |
| Hydroxyde de sodium à 10% | AGITATEUR : Rayneri |
| | PALE : Défloculeuse |
| | AGITATION : 680 tr/mn |
| | TEMPS : 25 min |

### III. ETUDE DE LA STABILITE

### Exemple 6: Stabilité physique de la composition par la mesure du seuil d'écoulement (en Pa.s⁻¹)

Les essais effectués sont des mesures de viscosité permettant de tracer des courbes appelées rhéogrammes, qui permettent à un gradient de vitesse donnée γ, de mesurer une contrainte de cisaillement τ, et à une contrainte de cisaillement donné τ, de mesurer un gradient de vitesse γ ("Initiation à la rhéologie" Gouarraze - Grossiord 1991; "La viscosité et sa mesure dans les pharmacopées"; L. Molle, Journal Pharma Belg. 1975, 30, 5-6, 597-619).

Par seuil d'écoulement, on entend la force nécessaire (contrainte de cisaillement minimum) pour vaincre les forces de cohésion de type Van der Waals et provoquer l'écoulement.

Le seuil d'écoulement (yield value τ0) est extrapolé soit visuellement (ordonnée à l'origine des rhéogrammes) soit par le calcul (application de modèles mathématiques).

Composition des gels utilisés :

**Gel aqueux à base de carbomer**

| **Constituants** | **%** |
|---|---|
| EAU PURIFIEE | QS 100% |
| E.D.T.A DISODIQUE | 0.10 |
| DOCUSATE DE SODIUM | 0.05 |
| SILICE (AEROSIL 200) | 0.02 |
| CARBONER (CARBOPOL 980) | 0.85 |
| **GLYCERINE** | **4.00** |
| PROPYLENE GLYCOL | 4.00 |
| POLOXAMER 124 | 0.20 |
| MICROEPONGES DE PEROXYDE DE BENZOYL | QS 2.5% BPO |
| ADAPALENE | 0.10 |
| SOUDE à10% | 2.00 qs pH 5 |

**Gel aqueux à base d'hydroxypropylcellulose**

| **Constituants** | **%** |
|---|---|
| EAU PURIFIEE | QS 100% |
| E.D.T.A DISODIQUE | 0.10 |
| SILICE (AEROSIL 200) | 0.02 |
| GLYCERINE | 4.00 |
| ARGILE (VEEGUM K) | 2.00 |
| GOMME DE XANTHANE (KELTROL T) | 0.50 |
| HYDROXYPROPYLCELLULOSE (NATROSOL HHX) | 1.50 |
| PROPYLENE GLYCOL | 4.00 |
| DOCUSATE DE SODIUM | 0.05 |
| POLOXAMER 124 | 0.20 |
| MICROEPONGES DE PEROXYDE DE BENZOYL | QS 2.5% BPO |
| ADAPALENE | 0.10 |

| | Exemple 2 selon l'invention | | Gel aqueux à base de Carbomer | | Gel aqueux à base d'hydroxypropyl cellulose et d'aluminium magnésium silicate | |
|---|---|---|---|---|---|---|
| **Température** | TA | T40°C | TA | T40°C | TA | T40°C |
| T initial | 137 | / | 111 | / | 170 | |
| T 1mois | 139 | 137 | 135 | 111 | 183 | 93 |
| T 2mois | 147 | 121 | / | / | 158 | 65 |
| T 3mois | 149 | 127 | 100 | 76 | 147 | 34 |

| | | | | | | |
|---|---|---|---|---|---|---|
| TA: Température ambiante T40°C: stockage à 40°C | | | | | | |

Le seuil d'écoulement de la composition selon l'invention est stable dans le temps et à température, contrairement aux deux autres exemples de gel aqueux dont la viscosité chute rapidement dans le temps aussi bien à température ambiante qu'à 40°C. Ces résultats démontrent une très bonne stabilité physique de la composition selon l'invention dans le temps contrairement aux compositions classiques de gels aqueux.

### Exempte 7 : Stabilité du peroxyde de benzoyle au cours du temps et en fonction de

### la température de stockage par la mesure de la quantité de peroxyde de benzoyle dans la composition (en pourcentage)

Les pourcentages en peroxyde de benzoyle (BPO) présentés dans le tableau ci-dessous ont été obtenus par la mesure de la concentration en peroxyde de benzoyle par iodométrie.
La quantité appropriée de composition est dissoute tout d'abord dans de l'eau purifiée puis dans de l'acétonitrile, et soumise à l'action d'une solution de iodure de potassium. Quand le iodure de potassium est ajouté, il se produit un changement de couleur du blanc au marron indiquant la présence du peroxyde de benzoyle dans la composition. L'iode libéré est titré utilisant une solution à 0.1 N de thiosulfate de sodium :

I₂ + 2 Na₂S₂O₃ → 2 Nal + Na₂S₄O₆

Les pourcentages de peroxyde de benzoyle inscrits dans le tableau ci-dessous correspondent au pourcentage de peroxyde de benzoyle mesuré dans le produit par rapport à la quantité théorique introduite.

| | Exemple 2 selon l'invention | | Gel aqueux à base de Carbomer | | Gel aqueux à base d'hydroxypropyl cellulose et d'aluminium magnésium silicate | |
|---|---|---|---|---|---|---|
| **Température** | **TA** | **T40°C** | **TA** | **T40°C** | **TA** | **T40°C** |
| % BPO à T initial | 100 | / | 94.3 | / | 101.7 | |
| % BPO à T1mois | Non réalisé | 102.5 | 95.7 | 90.3 | / | 94.9 |
| % BPO à T2mois | Non réalisé | 99.1 | 94.3 | 85.5 | / | 87.3 |
| % BPO à T3mois | 102.4 | 100.3 | 94 | 78.3 | 99.7 | 85.8 |

Dans la composition de l'exemple 2, le pourcentage de peroxyde de benzoyle dans le temps reste stable et équivalent à 100% aussi bien à température ambiante qu'à 40°C. Le peroxyde de benzoyle présent dans les deux autres exemples de gels aqueux de l'art antérieur se dégrade de façon significative dans le temps. Au bout de 3 mois, on peut observer une perte allant jusqu'à 6% à température ambiante et d'au moins 14% de peroxyde de benzoyle à T40°C.
Ces résultats démontrent que les compositions selon l'invention permettent une très bonne stabilité du peroxyde de benzoyle dans le temps et même à 40°C contrairement aux compositions classiques.

## Revendications

1. Composition comprenant, dans un milieu physiologiquement acceptable, au moins un rétinoide, du peroxyde de benzoyle dispersé et au moins un gélifiant pH-indépendant choisi parmi les polyacrylamides, les polymères acryliques couplés à des chaînes hydrophobes, les amidons modifiés et leurs mélanges.

2. Composition selon la revendication 1, **caractérisée en ce qu'**elle contient entre 0,1 à 15 % en poids par rapport au poids total de la composition de gélifiant pH-indépendant.

3. Composition selon l'une des revendications 1 ou 2, **caractérisée en ce que** le gélifiant pH-indépendant est un polyacrylamide.

4. Composition selon l'une des revendications 1 à 3, **caractérisée en ce qu'**elle contient entre 0,0001 et 20 % en poids par rapport au poids total de la composition de rétinoide.

5. Composition selon l'une des revendications 1 à 4, **caractérisée en ce que** le rétinoide est l'adapalène.

6. Composition selon l'une des revendications 1 à 5, **caractérisée en ce qu'**elle contient entre 0,0001 et 20 % en poids par rapport au poids total de la composition de peroxyde de benzoyle.

7. Composition selon l'une des revendications 1 à 6, **caractérisée en ce qu'**elle contient entre 2,5 et 5 % en poids par rapport au poids total de la composition de peroxyde de benzoyle.

8. Composition selon l'une des revendications 1 à 7, **caractérisée en ce que** le peroxyde de benzoyle est encapsulé ou libre.

9. Composition selon l'une quelconque des revendications précédentes, **caractérisée en ce qu'**elle contient un agent propénétrant.

10. Composition selon la revendication 9, **caractérisée en ce qu'**elle contient entre 0 et 20 % en poids par rapport au poids total de la composition d'agent propénétrant.

11. Composition selon la revendication 9 ou 10, **caractérisée en ce que** l'agent propénétrant est le propylène glycol.

12. Composition selon l'une quelconque des revendications précédentes, **caractérisée en ce qu'**elle contient un tensioactif liquide mouillant.

13. Composition selon la revendication 1 à 12, **caractérisée en ce qu'**elle contient entre 0 et 10 % en poids par rapport au poids total de la composition de tensioactif liquide mouillant.

14. Composition selon la revendication 12 ou 13, **caractérisée en ce que** le tensioactif liquide mouillant est un poloxamer.

15. Composition selon l'une des revendications 1 à 14, **caractérisée en ce qu'**elle comprend au moins un additif choisi parmi les séquestrants, les antioxydants, les filtres solaires, les conservateurs, les charges, les électrolytes, les humectants, les colorants, les bases et acides usuels, minéraux ou organiques, les parfums, les huiles essentielles, les actifs cosmétiques, les hydratants, les vitamines, les acides gras essentiels, les sphingolipides, les composés auto-bronzants, et les agents apaisants et protecteurs de la peau.

16. Composition selon l'une quelconque des revendications précédentes, **caractérisée en ce qu'**elle comprend dans l'eau, en poids par rapport au poids total de la composition :
- 2,50 % de peroxyde de benzoyle,
- 0,10 % d'adapalene,
- 0,10 % d'EDTA disodique,
- 4,00 % de glycérine,
- 4,00 % de propylène glycol,
- 0,05 %de docusate de sodium,
- 0,20 % de Poloxamer 124,
- 4,00 % de sodium acryloyldimethyltaurate copolymer & isohexadecane & polysorbate 80,
- qs pH 5 d'hydroxyde de sodium.

17. Composition selon l'une quelconque des revendications de 1 à 16 à titre de médicament.

18. Procédé de préparation de la composition selon l'une quelconque des revendications précédentes, **caractérisé en ce qu'**il comprend successivement les étapes suivantes réalisées à température ambiante :
a) la préparation d'une phase aqueuse comprenant de l'eau et éventuellement un chélatant et/ou un propénétrant et/ou un humectant ;
b) la préparation d'une phase active comprenant le mélange dans l'eau du rétinoide, du peroxyde de benzoyle et, éventuellement, d'un tensioactif liquide mouillant et/ou d'un propénétrant sous agitation ;
c) l'introduction de la phase active dans la phase aqueuse, sous agitation ; et
d) l'introduction du gélifiant sous agitation dans le mélange issu de l'étape c).

19. Procédé de préparation de la composition selon l'une quelconque des revendications 1 à 16, **caractérisé en ce qu'**il comprend successivement les étapes suivantes réalisées à température ambiante :
a) la préparation d'une phase aqueuse comprenant de l'eau et éventuellement un chélatant et/ou un propénétrant et/ou un humectant ;
b) la préparation de deux phases actives, l'une comprenant le mélange dans l'eau du rétinoide, l'autre comprenant le mélange dans l'eau du peroxyde de benzoyle et, éventuellement, d'un tensioactif liquide mouillant et/ou d'un propénétrant sous agitation ;
c) l'introduction des phases actives dans la phase aqueuse, sous agitation ; et
d) l'introduction du gélifiant sous agitation dans le mélange issu de l'étape c).

20. Utilisation d'une composition selon l'une quelconque des revendications de 1 à 16 pour la fabrication d'une préparation pharmaceutique destinée à prévenir ou à traiter les affections dermatologiques liées à des désordres de la différentiation et/ou la prolifération cellulaire et/ou de la kératinisation.

21. Utilisation d'une composition selon l'une quelconque des revendications de 1 à 16 pour la fabrication d'une préparation pharmaceutique destinée à prévenir ou à traiter les affections choisies parmi les acnés vulgaires, comédoniennes, polymorphes, rosacées, les acnés nodulokystiques, conglobata, les acnés séniles, l'acné solaire, médicamenteuse ou professionnelle et l'hidradenite supurative.

22. Utilisation d'une composition selon l'une quelconque des revendications de 1 à 16 pour fabriquer une préparation pharmaceutique destinée à prévenir ou à traiter les acnés vulgaires.

23. Utilisation cosmétique d'une composition selon l'une quelconque des revendications 1 à 16 pour le traitement des peaux à tendance acnéique, pour faire repousser les cheveux ou éviter leur chute, pour lutter contre l'aspect gras de la peau ou des cheveux ou dans le traitement des peaux physiologiquement sèches, ou pour prévenir et/ou lutter contre le vieillissement photo-induit ou chronologique.

## Claims

1. Composition comprising, in a physiologically acceptable medium, at least one retinoid, dispersed benzoyl peroxide and at least one pH-independent gelling agent chosen from polyacrylamides, acrylic polymers coupled to hydrophobic chains, and modified starches, and mixtures thereof.

2. Composition according to Claim 1, **characterized in that** it contains between 0.1% and 15% by weight relative to the total weight of the composition of pH-independent gelling agent.

3. Composition according to either of Claims 1 and 2, **characterized in that** the pH-independent gelling agent is a polyacrylamide.

4. Composition according to one of Claims 1 to 3, **characterized in that** it contains between 0.0001% and 20% by weight relative to the total weight of the composition of retinoid.

5. Composition according to one of Claims 1 to 4, **characterized in that** the retinoid is adapalene.

6. Composition according to one of Claims 1 to 5, **characterized in that** it contains between 0.0001% and 20% by weight relative to the total weight of the composition of benzoyl peroxide.

7. Composition according to one of Claims 1 to 6, **characterized in that** it contains between 2.5% and 5% by weight relative to the total weight of the composition of benzoyl peroxide.

8. Composition according to one of Claims 1 to 7, **characterized in that** the benzoyl peroxide is encapsulated or free.

9. Composition according to any one of the preceding claims, **characterized in that** it contains a pro-penetrating agent.

10. Composition according to Claim 9, **characterized in that** it contains between 0 and 20% by weight relative to the total weight of the composition of pro-penetrating agent.

11. Composition according to Claim 9 or 10, **characterized in that** the pro-penetrating agent is propylene glycol.

12. Composition according to any one of the preceding claims, **characterized in that** it contains a liquid wetting surfactant.

13. Composition according to Claims 1 to 12, **characterized in that** it contains between 0 and 10% by weight relative to the total weight of the composition of liquid wetting surfactant.

14. Composition according to Claim 12 or 13, **characterized in that** the liquid wetting surfactant is a poloxamer.

15. Composition according to one of Claims 1 to 14, **characterized in that** it comprises at least one additive chosen from sequestrants, antioxidants, sunscreens, preserving agents, fillers, electrolytes, humectants, dyes, common mineral or organic acids and bases, fragrances, essential oils, cosmetic active agents, moisturizers, vitamins, essential fatty acids, sphingolipids, self-tanning compounds, skin calmatives and skin-protecting agents.

16. Composition according to any one of the preceding claims, **characterized in that** it comprises, in water, by weight relative to the total weight of the composition:
- 2.50% benzoyl peroxide,
- 0.10% adapalene,
- 0.10% disodium EDTA,
- 4.00% glycerol,
- 4.00% propylene glycol,
- 0.05% sodium docusate,
- 0.20% Poloxamer 124,
- 4.00% sodium acryloyldimethyltaurate copolymer & isohexadecane & polysorbate 80,
- sodium hydroxide, qs pH 5.

17. Composition according to any one of Claims 1 to 16, as a medicinal product.

18. Process for preparing the composition according to any one of the preceding claims, **characterized in that** it successively comprises the following steps performed at room temperature:
a) the preparation of an aqueous phase comprising water and optionally a chelating agent and/or a pro-penetrating agent and/or a humectant;
b) the preparation of an active phase comprising the mixture in water of the retinoid, of benzoyl peroxide and, optionally, of a liquid wetting surfactant and/or of a pro-penetrating agent, with stirring;
c) the introduction of the active phase into the aqueous phase, with stirring; and
d) the introduction of the gelling agent into the mixture obtained from step c), with stirring.

19. Process for preparing the composition according to any one of Claims 1 to 16, **characterized in that** it successively comprises the following steps performed at room temperature:
a) the preparation of an aqueous phase comprising water and optionally a chelating agent and/or a pro-penetrating agent and/or a humectant;
b) the preparation of two active agents, one comprising the mixture in water of the retinoid, the other comprising the mixture in water of benzoyl peroxide and, optionally, of a liquid wetting surfactant and/or of a pro-penetrating agent, with stirring;
c) the introduction of the active phases into the aqueous phase, with stirring; and
d) the introduction of the gelling agent into the mixture obtained from step c), with stirring.

20. Use of a composition according to any one of Claims 1 to 16, for the manufacture of a pharmaceutical preparation for preventing or treating dermatological complaints associated with cell differentiation and/or proliferation disorders and/or keratinization disorders.

21. Use of a composition according to any one of Claims 1 to 16 for the manufacture of a pharmaceutical preparation for preventing or treating complaints chosen from common acne, comedones, polymorphs, rosacea, nodulocystic acne, conglobata, senile acne, solar acne, medication-related or occupational acne, and suppurative hidradenitis.

22. Use of a composition according to any one of Claims 1 to 16, to manufacture a pharmaceutical preparation for preventing or treating common acne.

23. Cosmetic use of a composition according to any one of Claims 1 to 16, for treating acne-prone skin, for regrowth of the hair or for preventing hair loss, for combating the greasy appearance of the skin or the hair, or in the treatment of physiologically dry skin, or for preventing and/or combating photoinduced or chronological ageing.

## Patentansprüche

1. Zusammensetzung, die in einem physiologisch akzeptablen Medium mindestens ein Retinoid, dispergiertes Benzoylperoxid und mindestens einen pH-unabhängigen Gelbildner enthält, der unter den Polyacrylamiden, Acrylpolymeren, die an hydrophobe Gruppen gebunden sind, modifizierten Stärken und deren Gemischen ausgewählt ist.

2. Zusammensetzung nach Anspruch 1, **dadurch gekennzeichnet, dass** sie 0,1 bis 15 Gew.-% pH-unabhängigen Gelbildner, bezogen auf das Gesamtgewicht der Zusammensetzung, enthält.

3. Zusammensetzung nach einem der Ansprüche 1 oder 2, **dadurch gekennzeichnet, dass** der pH-unabhängige Gelbildner ein Polyacrylamid ist.

4. Zusammensetzung nach einem der Ansprüche 1 bis 3, **dadurch gekennzeichnet, dass** sie 0,0001 bis 20 Gew.-% Retinoid, bezogen auf das Gesamtgewicht der Zusammensetzung, enthält.

5. Zusammensetzung nach einem der Ansprüche 1 bis 4, **dadurch gekennzeichnet, dass** das Retinoid das Adapalen ist.

6. Zusammensetzung nach einem der Ansprüche 1 bis 5, **dadurch gekennzeichnet, dass** sie 0,0001 bis 20 Gew.-% Benzoylperoxid, bezogen auf das Gesamtgewicht der Zusammensetzung, enthält.

7. Zusammensetzung nach einem der Ansprüche 1 bis 6, **dadurch gekennzeichnet, dass** sie 2,5 bis 5 Gew.-% Benzoylperoxid, bezogen auf das Gesamtgewicht der Zusammensetzung, enthält.

8. Zusammensetzung nach einem der Ansprüche 1 bis 7, **dadurch gekennzeichnet, dass** das Benzoylperoxid eingekapselt ist oder frei vorliegt.

9. Zusammensetzung nach einem der vorhergehenden Ansprüche, **dadurch gekennzeichnet, dass** sie einen penetrationsfördernden Stoff enthält.

10. Zusammensetzung nach Anspruch 9, **dadurch gekennzeichnet, dass** sie 0 bis 20 Gew.-% penetrationsfördernden Stoff, bezogen auf das Gesamtgewicht der Zusammensetzung, enthält.

11. Zusammensetzung nach Anspruch 9 oder 10, **dadurch gekennzeichnet, dass** der penetrationsfördernde Stoff das Propylenglycol list.

12. Zusammensetzung nach einem der vorhergehenden Ansprüche, **dadurch gekennzeichnet, dass** sie einen benetzenden flüssigen grenzflächenaktiven Stoff enthält.

13. Zusammensetzung nach Anspruch 1 bis 12, **dadurch gekennzeichnet, dass** sie 0 bis 10 Gew.-% benetzenden flüssigen grenzflächenaktiven Stoff, bezogen auf das Gesamtgewicht der Zusammensetzung, enthält.

14. Zusammensetzung nach Anspruch 12 oder 13, **dadurch gekennzeichnet, dass** der benetzende flüssige grenzflächenaktive Stoff ein Poloxamer ist.

15. Zusammensetzung nach einem der Ansprüche 1 bis 14, **dadurch gekennzeichnet, dass** sie mindestens einen Zusatzstoff enthält, der unter den Maskierungsmitteln, Antioxidantien, Sonnenschutzfiltern, Konservierungsmitteln, Füllstoffen, Elektrolyten, Feuchthaltemitteln, Farbmitteln, üblichen anorganischen oder organischen Basen und Säuren, Parfums, etherischen Ölen, kosmetischen Wirkstoffen, Hydratisierungsmitteln, Vitaminen, essentiellen Fettsäuren, Sphingolipiden, Selbstbräunungsmitteln, beruhigenden Stoffen und Hautschutzmitteln ausgewählt ist.

16. Zusammensetzung nach einem der vorhergehenden Ansprüche, **dadurch gekennzeichnet, dass** sie in Wasser, bezogen auf das Gesamtgewicht der Zusammensetzung, enthält:
- 2,50 % Benzoylperoxid,
- 0,10 % Adapalen,
- 0,10 % Dinatrium-EDTA,
- 4,00 % Glycerin,
- 4,00 % Propylenglycol,
- 0,05 % Natriumdocusat,
- 0,20 % Poloxamer 124,
- 4,00 % Sodium Acryloyldimethyltaurate Copolymer & Isohexadecane & Polysorbate 80,
- qs pH 5 Natriumhydroxid.

17. Zusammensetzung nach einem der Ansprüche 1 bis 16 als Arzneimittel.

18. Verfahren zur Herstellung einer Zusammensetzung nach einem der vorhergehenden Ansprüche, **dadurch gekennzeichnet, dass** bei Raumtemperatur die folgenden, nacheinander ausgeführten Schritte durchgeführt werden:
a) Herstellen einer wässrigen Phase, die Wasser und gegebenenfalls einen Chelatbildner und/oder einen penetrationsfördernden Stoff und/oder ein Feuchthaltemittel enthält;
b) Herstellen einer aktiven Phase, die in Wasser das Gemisch aus Retinoid und Benzoylperoxid und gegebenenfalls einen benetzenden flüssigen grenzflächenaktiven Stoff und/oder einen penetrationsfördernden Stoff enthält, unter Rühren;
c) Einbringen der aktiven Phase in die wässrige Phase unter Rühren; und
d) Einarbeiten des Gelbildners in das Gemisch aus Schritt c) unter Rühren.

19. Verfahren zur Herstellung einer Zusammensetzung nach einem der Ansprüche 1 bis 16, **dadurch gekennzeichnet, dass** es die bei Umgebungstemperatur durchgeführten, aufeinander folgenden Schritte aufweist:
a) Herstellung einer wässrigen Phase, die Wasser und gegebenenfalls einen Chelatbildner und/oder einen penetrationsfördernden Stoff und/oder ein Feuchthaltemittel enthält;
b) Herstellung von zwei aktiven Phasen, eine, die das Mischen des Retinoids in Wasser umfasst, und eine weitere, die das Mischen von Benzoylperoxid und gegebenenfalls eines benetzenden flüssigen grenzflächenaktiven Stoffes und/oder eines penetrationsfördernden Stoffes unter Rühren umfasst;
c) Einbringen der aktiven Phasen in die wässrige Phase unter Rühren; und
d) Einarbeiten des Gelbildners in das Gemisch aus Schritt c) unter Rühren.

20. Verwendung einer Zusammensetzung nach einem der Ansprüche 1 bis 16 für die Herstellung eines pharmazeutischen Präparats, das dazu vorgesehen ist, dermatologischen Erkrankungen vorzubeugen, die mit Störungen der Differenzierung und/oder der Proliferation der Zellen und/oder der Keratinisierung zusammenhängen, oder sie zu behandeln.

21. Verwendung einer Zusammensetzung nach einem der Ansprüche 1 bis 16 für die Herstellung eines pharmazeutischen Präparats, das dazu vorgesehen ist, Störungen vorzubeugen, die unter Acne vulgaris, Acne comedonica, polymorpher Acne, Acne rosaceae, nodulocystischer Acne, Acne conglobata, Acne senilis, Acne solaris, Acne medicamentosa oder Acne professionalis und Hidradenitis suppurativa ausgewählt sind, oder sie zu behandeln.

22. Verwendung einer Zusammensetzung nach einem der Ansprüche 1 bis 16 für die Herstellung eines pharmazeutischen Präparats, das dazu vorgesehen ist, Acne vulgaris vorzubeugen oder zu behandeln.

23. Kosmetische Verwendung einer Zusammensetzung nach einem der Ansprüche 1 bis 16 für die Behandlung von Haut mit Aknetendenz, für den Haarwuchs oder zur Vermeidung von Haarausfall, zur Bekämpfung des fettigen Aussehens der Haut oder der Haare oder für die Behandlung von physiologisch trockener Haut oder um der lichtinduzierten oder chronologischen Alterung vorzubeugen und/oder sie zu bekämpfen.
